# EUROPÄISCHE PATENTANMELDUNG

(11) **EP 3 009 165 A1**
(43) Veröffentlichungstag der Anmeldung: **20.04.2016**
(21) Anmeldenummer: 15184816.5
(22) Anmeldetag: 11.09.2015
(51) Int. Cl.: A61N 1/05

(54) **IMPLANTIERBARE ELEKTRISCHE LEITUNG**

(30) Priorität: 16.10.2014 US 201462064486 P
(71) Anmelder: BIOTRONIK SE & Co. KG, 12359 Berlin (DE)
(72) Erfinder: Weitzig, Pierre, 54666 Irrel (DE); Friedrich, Michael, 14532 Kleinmachnow (DE); Rump, Jens, 12049 Berlin (DE)
(74) Vertreter: Galander, Marcus

(57) **Zusammenfassung**

Die Erfindung betrifft eine implantierbare elektrische Leitung (20) mit wenigstens einem helixartig gewendelten elektrischem Leiter (26), einer mit dem elektrischen Leiter elektrisch verbundenen elektrisch leitenden Hülle (22; 24) und einem elektrischen Filter (40). Der elektrische Filter ist in Längsrichtung der elektrischen Leitung (20) gesehen zwischen einem proximalen (20p) und einem distalen Längsabschnitt (20d) einer von dem helixartig gewendelten elektrischen Leiter (26) gebildeten Helix sowie in radialer Richtung der elektrischen Leitung (20) gesehen innerhalb der elektrisch leitenden Hülle (22; 24) angeordnet.

## Beschreibung

Die Erfindung betrifft ein permanent oder temporär implantierbares Gerät mit einem langgestreckten elektrischen Leiter, nämlich eine implantierbare elektrische Leitung mit einem elektrischen Filter zur Vermeidung von radiofrequenzinduzierten Erwärmungen.

Solche Geräte, beispielsweise Elektrodenleitungen für die Elektrostimulation, haben den Nachteil, dass sich ihr elektrischer Leiter in einem Kernspintomografen erwärmen kann, weil die im Kernspintomografen herrschenden wechselnden Magnetfelder in dem elektrischen Leiter nicht unbeachtliche elektrische Ströme induzieren. Deshalb können Herzschrittmacherpatienten heutzutage in der Regel nicht oder nur eingeschränkt in einem Kernspintomografen untersucht werden.

An implantierbaren Herzschrittmachern oder Defibrillatoren sind nämlich typischerweise wenigstens eine Stimulationselektrodenleitung angeschlossen, die an ihrem proximalen, zum Anschluss an den Herzschrittmacher oder Defibrillator vorgesehenen Ende einen standardisierten elektrischen Anschluss aufweist und an ihrem distalen, zur Platzierung im Herzen vorgesehenen Ende einen oder mehrere Elektrodenpole aufweist. Ein solcher Elektrodenpol dient zur Abgabe elektrischer Impulse an das Gewebe (Myokard) des Herzens oder zum Abfühlen elektrischer Felder, um im Rahmen des sogenannten Sensings eine Aktivität eines Herzens abfühlen zu können. Zu diesen Zwecken bilden Elektrodenpole typischerweise elektrisch leitende Oberflächenabschnitte einer Elektrodenleitung. Elektrodenpole sind typischerweise als Ringelektrode in Form eines Rings um die Elektrodenleitung oder in Form einer Spitzen- oder Tippelektrode am distalen Ende der Elektrodenleitung vorgesehen. Die Elektrodenpole sind über eine oder mehrere elektrische Leiter mit Kontakten des elektrischen Anschlusses der Elektrodenleitung an deren proximalem Ende elektrisch leitend verbunden. Somit verlaufen zwischen den Kontakten des elektrischen Anschlusses die Elektrodenleitungen an deren proximalen Ende und den Elektrodenpolen am distalen Ende der Elektrodenleitung ein oder mehrere elektrische Leiter, die einen oder mehrere der Elektrodenpole mit einem oder mehreren der Kontakte elektrisch verbinden. Diese elektrischen Leiter können einerseits zur Übertragung von Stimulationsimpulsen zu den Elektrodenpolen und andererseits zur Übertragung mittels der Elektrodenpole aufgenommener elektrischer Signale zum proximalen Ende der Elektrodenleitung genutzt werden und werden im Verlauf der weiteren Beschreibung auch jeweils als Funktionsleitung bezeichnet. Solche Funktionsleitungen sind für die Funktionen der jeweiligen Elektrodenleitung erforderliche elektrische Leiter und als solche der Gefahr ausgesetzt, dass in ihnen durch äußere Wechselmagnetfelder elektrische Ströme induziert werden, die beispielsweise zu einer unerwünschten Erwärmung der Funktionsleitungen oder der mit ihr verbundenen Elektrodenpole führen können oder die zur Abgabe entsprechender Ströme über die Elektrodenpole an umgebendes Gewebe und damit zu einer Erwärmung des umgebenden Gewebes führen können.

Implantierbare Leitungen, wie sie u.a. als Elektrodenleitungen für Herzschrittmacher verwendet werden, wirken bei Einstrahlung elektromagnetischer Wellen ähnlich einer Antenne und können die aufgenommene Energie in Wärme umwandeln. Die Erhitzung tritt vorzugsweise an Leitungsenden auf, was zur Gewebeschädigung führen kann. Durch einen mit dem Elektrodenpol elektrisch in Serie geschalteten, mechanisch entweder proximal oder distal dazu befindlichen Bandstoppfilter (oder anderer elektrischer Filter) werden elektrische Wellen im Radiofrequenzbereich reflektiert und somit die Erwärmung des Gewebes am Elektrodenpol verringert.

Für Elektrodenleitungen mit Coaxialwendeln, bei denen die Funktionsleiter jeweils eine einzelne Helix mit jeweils unterschiedlichem Durchmesser bilden, ist es bekannt, einen kapazitativ gekoppelten Shunt oder jeweils einen Bandstoppfilter proximal zur Elektrode vorzusehen.

Für mehrpolige Elektrodenleitungen die mehrere Elektrodenpole aufweisen, welche jeweils mit eigenem Funktionsleiter mit entsprechenden Kontakten des elektrischen Anschlusses der Elektrodenleitung verbunden sind, ist außerdem eine Leitungskonstruktion bekannt, die als coradial bezeichnet wird. Hierbei sind die einzelnen Funktionsleiter voneinander isoliert zu einer mehrgängigen Helix gewickelt, bei der die einzelnen Leiter zueinander kongruente Helices mit einander gleichem Durchmesser und gleicher Steigung bilden, die so ineinander greifen, dass die Windungen der Einzelhelices wie Gewindegänge einer mehrgängigen Schraube in Längsrichtung der Helix periodisch aufeinander folgen und so eine coradiale Leitungswendel bilden. Insbesondere für coradiale Elektrodenleitungen (also Elektrodenleitungen mit coradialer Leitungswendel) ist bisher keine befriedigende Lösung für die Realisierung von Bandstopp-Filtern bekannt.

Der Erfindung liegt die Aufgabe zugrunde, eine verbesserte implantierbare Leitung mit einem elektrischen Filter zu schaffen.

Erfindungsgemäß wird diese Aufgabe durch eine implantierbare elektrische Leitung mit wenigstens einem helixartig gewendelten elektrischem Leiter, einer mit dem elektrischen Leiter elektrisch verbundenen elektrisch leitenden Hülle und einem elektrischen Filter gelöst, wobei der elektrische Filter in Längsrichtung der elektrischen Leitung gesehen zwischen einem proximalen und einem distalen Längsabschnitt einer von dem helixartig gewendelten elektrischem Leiter gebildeten Helix sowie in radialer Richtung der elektrischen Leitung gesehen innerhalb der elektrisch leitenden Hülle angeordnet ist.

Vorzugsweise ist die implantierbare elektrische Leitung eine mehrpolige Elektrodenleitung mit mehreren elektrisch leitenden Hüllen als Elektrodenpolen, die jeweils mit einem elektrischen Leiter elektrisch verbunden sind, wobei die elektrischen Leiter eine coradiale Leitungswendel bilden.

Die Erfindung schließt die Erkenntnis ein, dass eine der effektivsten Maßnahmen gegen MRT-Erwärmung ein Bandstoppfilter ist, ein Element, das elektrisch zwischen der Zuleitung und dem dazugehörigem Elektrodenpol geschaltet werden muss. Doch bei einer Coradial-Elektrodenleitung fehlt der Platz (besonders radial), um einen solchen Filter auch mechanisch unmittelbar proximal zum jeweiligen Elektrodenpol unterzubringen. Außerdem wird evtl. für jeden der bis zu 4 Elektrodenpole jeweils ein Filter benötigt. Gemäß der Erfindung werden die jeweiligen elektrischen Filter deshalb axial verlagert an Stellen untergebracht, die mehr Platz bieten ohne die Flexibilität oder sonstige Funktionseigenschaften der Elektrode zu beeinträchtigen. Alternativ zu einem Bandstoppfilter kann ein anderer elektrischer Filter, z.B. ein Tiefpassfilter, verwendet werden.

Bei einer mehrpoligen Elektrodenleitung, die wenigstens einen am weitesten distal gelegenen (distalsten), einen davon verschiedenen am weitesten proximal gelegenen (proximalsten) und wenigstens einen dazwischen gelegenen mittleren Elektrodenpol aufweist, ist es insbesondere bevorzugt, wenn ein jeweiliger elektrischer Filter für den mittleren und/oder den distalsten Elektrodenpol innerhalb der elektrisch leitenden Hülle, die den jeweils benachbarten, jeweils nächst-proximaleren Elektrodenpol bildet, angeordnet ist. D.h. ein jeweiliger elektrischer Filter für einen jeweiligen Elektrodenpol ist zumindest im Falle eines mittleren Elektrodenpols nicht innerhalb der zugehörigen elektrisch leitenden Hülle sondern innerhalb einer nächstgelegenen, benachbarten elektrisch leitenden Hülle angeordnet. Dies erleichtert das Kontaktieren eines Filters für einen Elektrodenpol und der zugehörigen elektrisch leitenden Hülle.

Vorzugsweise ist der elektrische Filter für den proximalsten Elektrodenpol proximal der dazugehörigen elektrisch leitenden Hülle angeordnet, und zwar vorzugsweise in einem Bereich der Elektrodenleitung, der steifer und/oder von größerem Durchmesser sein darf als der distale Bereich, ohne die sonstigen Funktionen der Elektrodenleitung zu beeinträchtigen.

Vorzugsweise ist der elektrische Filter für den distalsten Elektrodenpol distal zu diesem Elektrodenpol, z.B. in einem flexiblen Tip-Bereich der Elektrodenleitung, angeordnet.

Eine jeweilige elektrisch leitende Hülle bildet vorzugsweise jeweils eine Ringelektrode der Elektrodenleitung.

Insgesamt ergibt sich eine bevorzugte Ausführungsform einer mehrpoligen Elektrodenleitung mit coradialem Zuleitungsaufbau mit folgender Anordnung der elektrischen Filter:
- der Filter wird unter der dazugehörigen Ringelektrode untergebracht
   oder

- der Filter für die mittleren und/oder distalste Ringelektroden sind unter der benachbarten, jeweils nächst-proximaleren Ringelektrode untergebracht,
- der Filter für die proximalste Ringelektrode ist proximal dazu untergebracht, in einem Bereich der Elektrode, der steifer und/oder von größerem Durchmesser sein darf als der distale Bereich, ohne die sonstigen Funktionen der Elektrode zu beeinträchtigen, und
- der Filter für den distalsten Elektrodenpol ist distal zu diesem Elektrodenpol, in einem flexiblen Tip-Bereich der Elektrodenleitung, angeordnet.

Für den jeweiligen elektrischen Filter sind folgende Ausführungsvarianten bevorzugt:
Der Filter weist eine Spule aus isoliertem Draht auf, der mit einer ungeraden Anzahl an Lagen um einen hohlen, zylindrischen Kern helixförmig gewickelt ist.
Der Filter weist eine Spule aus isoliertem Draht auf, der mit einer geraden Anzahl an Lagen um einen hohlen, zylindrischen Kern helixförmig gewickelt ist.
Der Filter weist einen einlagig gewickelten, nackten oder isolierten Draht auf, und optional ein zusätzliches kapazitives Element.
Der Filter weist eine spiralförmig um einen hohlen, zylindrischen Kern gewickelte Spule aus einseitig metallisierter Folie auf. Die Folie ist vorzugsweise eine Kunststofffolie.

Vorzugsweise ist der elektrische Filter ein Bandstopp- oder Tiefpass-Filter.

Vorzugsweise ist ein jeweiliger elektrischer Filter mit aufgetrennten Leitungsenden eines aufgetrennten Leiters einer Coradialwendelzuleitung elektrisch verbunden und andere Leiter der Coradialwendelzuleitung sind im Bereich des elektrischen Filters nicht aufgetrennt, sondern ununterbrochen an dem elektrischen Filter vorbeigeführt.

Erfindungsgemäß ist auch ein Herstellungsverfahren für eine Elektrodenleitung mit wenigstens einem Elektrodenpol und einem elektrischen Filter vorgesehen, welches die folgenden Schritte aufweist:
- Aufdrehen der Coradialwendelzuleitung am Ort des Elektrodenpols,
- Auftrennen eines Leiters der Coradialwendelzuleitung, so dass aufgetrennte Leitungsenden entstehen,
- Befreien der aufgetrennten Leitungsenden von Isolation,
- Einsetzen eines elektrischen Filters dort, wo die Coradialwendelzuleitung aufgedreht ist,
- elektrisches Kontaktieren des elektrischen Filters mit den von Isolation befreiten Enden des aufgetrennten Leiters,
- Aufschieben einer einen späteren Elektrodenpol bildenden elektrisch leitenden Hülle, so dass sich der elektrische Filter schließlich innerhalb der elektrisch leitenden Hülle befindet,
- Kontaktieren der elektrisch leitenden Hülle,
- Isolieren offenliegender Drähte und Kontaktstellen im Bereich des Elektrodenpols.

Mit einer erfindungsgemäßen Elektrodenleitung lassen sich folgende Vorteile erzielen:
Es wird Platz geschaffen für die Unterbringung eines Bandstoppfilters unter Beibehaltung der günstigen Flexibilität in anderen Abschnitten des Elektrodenkörpers

Eine Platzierung eines distalsten elektrischen Filters im distalen Ende der Elektrodenleitung bietet sich an, weil ohnehin ein flexibles, distales Ende ohne Elektrodenpole benötigt wird, um eine Phrenicus-Nervenstimulation zu vermeiden, die sonst bei einer zu distalen Position der Ringelektroden vorkommen kann.

Die Erfindung soll nun anhand von Ausführungsbeispielen mit Bezug auf die Figuren näher erläutert werden. Die Figuren zeigen Folgendes:
- Fig. 1: zeigt als implantierbare medizinische Geräte einen implantierbaren Herzstimulator 10 und eine daran angeschlossene implantierbare Elektrodenleitung 20.
- Fig. 2: zeigt Details einer Coradialwendelzuleitung vor Einbau des Filters.
- Fig. 3: zeigt Details einer Coradialwendelzuleitung nach Einbau des Filters.
- Fig. 4: zeigt ein distales Ende einer Coradialwendelzuleitung.
- Fig. 5: zeigt ein distales Ende einer Coradialwendelzuleitung.
- Fig. 6: zeigt ein Detail einer Coradialwendelzuleitung.
- Fig. 7: ein distales Ende einer erfindungsgemäßen zweipoligen Elektrodenleitung.
- Fig. 8: Details einer Coradialwendelzuleitung vor Einbau des Filters.
- Fig. 9: Details einer Coradialwendelzuleitung nach Einbau des Filters.
- Fig. 10: zeigt eine Coradialwendelzuleitung mit aufgezogener isolierender Hülle.
- Fig. 11: Details einer Coradialwendelzuleitung vor Einbau des Filters.
- Fig. 12: Details einer Coradialwendelzuleitung nach Einbau des Filters.
- Fig. 13: zeigt eine Coradialwendelzuleitung mit aufgeschobener elektrisch leitender Hülle.
- Fig. 14: Detail einer fertiggestellten Coradialwendelzuleitung.

Der implantierbare Herzstimulator 10 kann ein Herzschrittmacher oder ein Kardioverter/Defibrillator (ICD) sein. Im dargestellten Ausführungsbeispiel ist der Herzstimulator 10 ein ventrikulärer Herzschrittmacher und Defibrillator. Andere bekannte Herzstimulatoren sind Zweikammerherzschrittmacher zur Stimulation des rechten Atriums und des rechten Ventrikels oder biventrikuläre Herzschrittmacher, die zusätzlich zum rechten Ventrikel auch den linken Ventrikel stimulieren können.

Derartige Stimulatoren besitzen typischerweise ein Gehäuse 12, das im Regelfall aus Metall besteht und somit elektrisch leitend ist und als großflächiger Elektrodenpol dienen kann. An der Außenseite des Gehäuses 12 ist typischerweise ein Anschlussgehäuse 14 befestigt, das auch als Header bezeichnet wird. Ein derartiger Header weist typischerweise Kontaktbuchsen zur Aufnahme von Steckkontakten auf. Die Kontaktbuchsen besitzen elektrische Kontakte 16, die über entsprechende Leiter mit einer in dem Gehäuse 12 des Herzstimulators 10 angeordneten Elektronik verbunden sind.

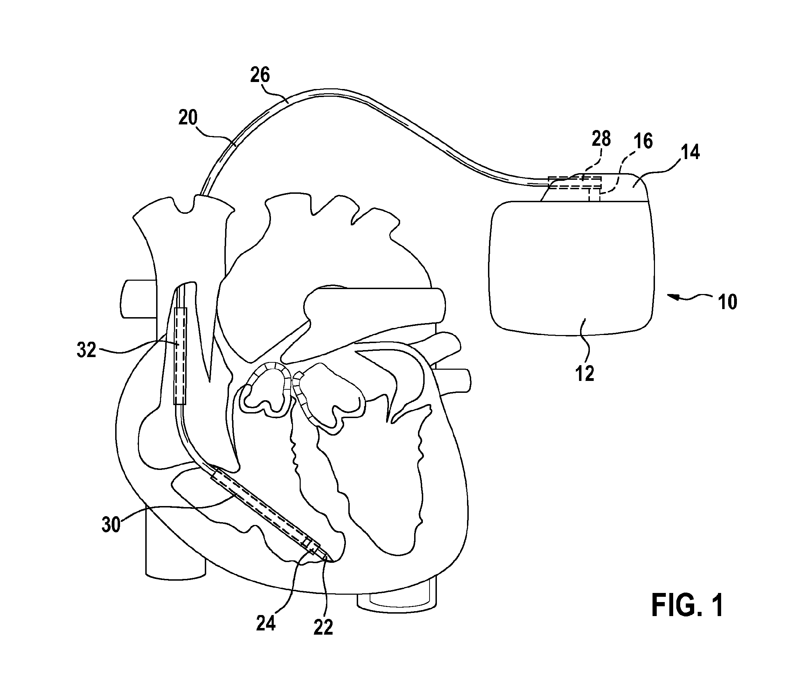

Die Elektrodenleitung 20 stellt im Sinne dieser Erfindung ebenfalls ein implantierbares medizinisches Gerät im Allgemeinen und eine implantierbare elektrische Leitung im Speziellen dar. Am distalen Ende der Elektrodenleitung 20 sind in an sich bekannter Manier Elektrodenpole in Form einer Spitzen- oder Tipelektrode 22 und einer in deren Nähe angeordneten Ringelektrode 24 angeordnet. Die Elektrodenpole 22 und 24 sind so ausgebildet, dass sie je nach Funktion eines Herzstimulators, an den die Elektrodenleitung 20 angeschlossen ist, zum Abfühlen elektrischer Potenziale des Herzgewebes (Myokards) dienen oder zur Abgabe elektrischer Signale, beispielsweise zur Abgabe von Stimulationsimpulsen an das sie umgebende Herzgewebe, ausgebildet sind. Fig. 1 zeigt, wie sich die Elektrodenpole, also die Tipelektrode 22 und die Ringelektrode 24, im Anwendungsfall die Elektrodenleitung 20, im Apex eines rechten Ventrikels eines Herzens befinden.

Sowohl die Tipelektrode 22 als auch die Ringelektrode 24 sind über jeweils wenigstens einen elektrischen Leiter 26.1 bzw. 26.2 mit einem Steckkontakt 28 am proximalen Ende der Elektrodenleitung 20 elektrisch verbunden. Die elektrischen Leiter bilden gemeinsam eine Coradialwendelzuleitung 26. Der Steckkontakt 28 besitzt elektrische Kontakte, die mit den elektrischen Kontakten 16 der Kontaktbuchse im Anschlussgehäuse 14 des implantierbaren Herzstimulators korrespondieren. Die elektrischen Leiter 26 in der Elektrodenleitung 20 können als annähernd gestreckte Seilzugleiter oder als helixförmig gewendelte Leiter ausgebildet sein. Solche Leiter, die funktionale Elektrodenpole mit elektrischen Kontakten des Steckkontaktes am proximalen Ende der Elektrodenleitung 20 elektrisch leitend verbinden werden im Rahmen dieses Textes als Funktionsleiter bezeichnet, da sie beispielsweise der Therapie dienende elektrische Signale von Steckkontakt zum jeweiligen Elektrodenpol übertragen oder abgefühlte elektrische Potentiale repräsentierende Signale vom jeweiligen Elektrodenpol zum Steckkontakt führen und somit der elementaren Funktion des medizinischen Gerätes dienen.

Die elektrischen Leiter 26, die die Elektrodenpole 22 bzw. 24 mit den elektrischen Kontakten des Steckers 28 der Elektrodenleitung 20 verbinden, sind über den größten Teil ihrer Länge von einer isolierenden Hülle umgeben, so dass ein elektrischer Kontakt zum Gewebe des Herzens gezielt über die Elektrodenpole zustande kommt.

Neben den Elektrodenpolen 22 und 24, die typischerweise der (in diesem Fall ventrikulären) Stimulation des Herzgewebes dienen, weist die Elektrodenleitung 20 auch noch zwei großflächigere Elektrodenpole 30 und 32 auf, die als Defibrillationselektroden dienen und von wenigstens einem blank liegendem helixartig gewendelten Draht gebildet sind.

Es sei darauf hingewiesen, dass die Erfindung im Rahmen dieses Ausführungsbeispiels anhand eines rechtsventrikulären Herzschrittmachers und Defibrillators erläutert wird. Als medizinisches Gerät im Sinne der Erfindung kann grundsätzlich aber beispielsweise auch eine Ablationselektrodenleitung dienen, die im Anwendungsfall ebenfalls bis ins Herz eines Patienten hineinragt und die von einem außerhalb des Patienten angeordneten Gerät gesteuert wird und hierzu mit diesem verbunden ist.

Fig. 1 zeigt eine Elektrodenleitung 20 mit zwei Elektrodenpolen 22 und 24, von denen der Elektrodenpol 24 eine Ringelektrode ist. Abweichend von dem Abweichungsbeispiel in Fig. 1 betrifft die vorliegende Erfindung insbesondere auch Elektrodenleitungen, die mehrere Elektrodenpole in Form von Ringelektroden aufweisen.

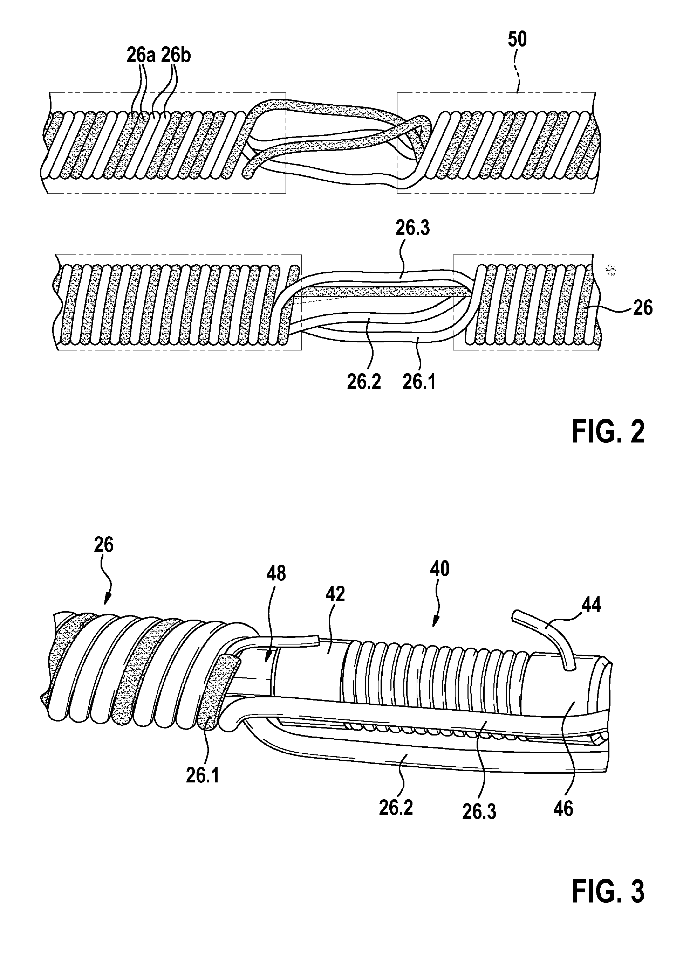

Zur Vermeidung der eingangs beschriebenen Problematik, insbesondere zur Vermeidung einer Erwärmung der Elektrodenpole, ist vorzugsweise jedem Elektrodenpol ein elektrischer Filter 40 zugeordnet, der vorzugsweise ein Bandstopp- oder Tiefpass-Filter ist. Die Figuren 2 bis 14 zeigen anhand von Beispielen, wie insbesondere bei coradialen Elektrodenleitungen solche elektrischen Filter 40 angeordnet und mit einem jeweiligen Elektrodenpol elektrisch verbunden werden können.

Für den jeweiligen elektrischen Filter sind die vorher erwähnten Ausführungsvarianten bevorzugt. Typischerweise enthält ein elektrischer Filter einen metallischen Draht oder eine metallische Folie, der oder die so aufgewickelt ist, dass eine dadurch entstehende Induktivität und eine (parasitäre) Kapazität einen Bandstopp- oder Tiefpass-Filter bilden, dessen Resonanz- oder Sperrfrequenz nahe der Frequenz eines zu erwartenden störenden elektromagnetischen Feldes liegt, insbesondere bei der Frequenz solcher elektrischen Felder, wie sie von einem Kernspintomograph(MRT)-Gerät erzeugt werden. Falls der elektrische Filter 40 eine Spule aus einseitig metallisierter Folie aufweist, die um einen hohlen zylindrischen Kern spiralförmig gewickelt ist, kann ein zusätzliches kapazitives Element elektrisch parallel zur Induktivität geschaltet werden, falls die parasitäre Kapazität durch die von der metallisierten Folie gebildete Kapazität nicht ausreicht, um eine gewünschte Resonanzfrequenz zu erzielen.

Alle Varianten des elektrischen Filters besitzen ein zentrales Lumen, durch das ein Mandrin oder Führungsdraht geschoben werden kann. Ein jeweiliger elektrischer Filter muss so in einen Elektrodenkörper der Elektrodenleitung eingebaut werden, dass der elektrische Filter vor Mehrbelastung, zum Beispiel vor zyklischer Biegung, geschützt ist.

Um einen elektrischen Filter 40 so in eine Elektrodenleitung, insbesondere eine coradiale Elektrodenleitung, einzubauen, dass deren Durchmesser und Steifigkeit darunter nicht leidet und gleichzeitig der elektrische Filter geschützt ist, ist es vorgesehen, einen jeweiligen elektrischen Filter 40 unter einer beispielsweise einen Ringelektrodenpol 24 bildenden elektrisch leitenden Hülle anzuordnen. Dazu kann - wie in Fig. 2 dargestellt - eine Coradialwendelzuleitung 26 an einem Ort, an dem ein elektrischer Filter 40 montiert werden soll, so aufgedreht werden, dass zunächst die Helixform der Coradialwendelzuleitung an diesem Ort unterbrochen ist, ohne dass die elektrischen Leiter selbst durchtrennt sind (siehe Fig. 2).

In Fig. 2 ist eine Coradialwendelzuleitung zu sehen, bei der zwei Doppelleitungen - eine helle und eine dunkle - zu einer Coradialwendel verbunden sind. Bei dieser Coradialwendelzuleitung kontaktieren die beiden dunklen Leiter 26a einen ersten Elektrodenpol und die beiden hellen Leiter 26b einen zweiten Elektrodenpol. Einer der beiden Elektrodenpole 24 befindet sich im fertiggestellten Zustand der Elektrodenleitung an dem in Fig. 2 dargestellten Ort, an dem die Helixform der Coradialwendelzuleitung unterbrochen ist. Fig. 2 zeigt kein Abbild der fertigen Elektrodenleitung 20.

Fig. 3 zeigt, wie nach Aufdrehen der Coradialwendelzuleitung ein elektrischer Filter 40 in dem aufgedrehten Abschnitt der Coradialwendelzuleitung angeordnet werden kann. Um den elektrischen Filter 40 zu kontaktieren, wird einer der Leiter 26 (der in Fig. 3 dunkel dargestellt ist) unterbrochen und ein abisoliertes Ende dieses Leiters mit einer proximalen Anschlusshülle 42 des elektrischen Filters 40 zum Beispiel durch Schweißen verbunden. Mit einem weiteren Draht 44 kann eine zweite Anschlusshülle 46 am anderen Ende des elektrischen Filters 40 mit einer einen Elektrodenpol bildenden, elektrisch leitenden Hülle (in Fig. 3 nicht dargestellt) verbunden werden. Die in Fig. 3 nicht dargestellte elektrische Hülle wird anschließend über den in Fig. 3 dargestellten elektrischen Filter 40 geschoben und mit dem Draht 44 elektrisch verbunden. Mit diesem Ausführungsbeispiel befindet sich schließlich der elektrische Filter 40 innerhalb derjenigen elektrisch leitenden Hülle, die jenen Elektrodenpol bildet, dem der elektrische Filter 40 zugeordnet ist.

Zum Einsetzen des elektrischen Filters 40 ist es vorteilhaft, einen Hilfskörper 48, beispielsweise einen Kunststoffschlauch (zum Beispiel aus Polyimid), in das zentrale Lumen der Coradialwendelzuleitung und des elektrischen Filters 40 einzuführen, um so den elektrischen Filter 40 an seinem Ort zu halten und so lange zu stabilisieren, bis die Elektrodenleitung 20 fertiggestellt ist. Der Kunststoffschlauch 48 kann danach an seinem Ort verbleiben oder entfernt werden.

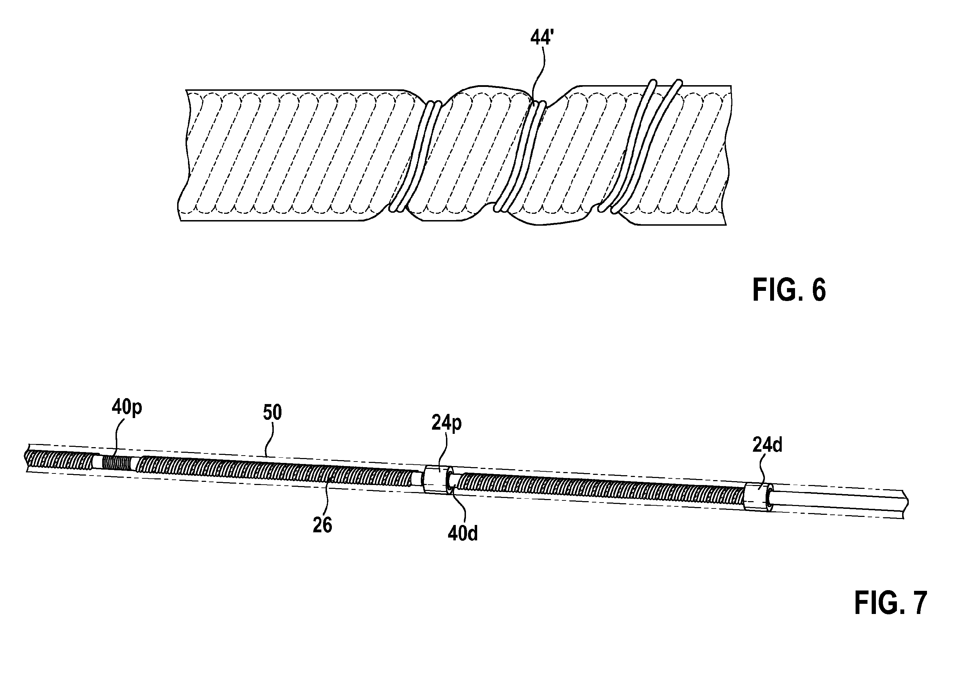

Da das Verbinden einer elektrisch leitenden Hülle mit einem entsprechend kurzen Draht, wie den Draht 44, innerhalb der elektrisch leitenden Hülle schwierig ist, kann ein jeweiliger elektrisch leitender Filter 40 auch innerhalb einer benachbarten elektrisch leitenden Hülle untergebracht werden, also nicht unter derjenigen elektrisch leitenden Hülle, die den Elektrodenpol bildet, dem der elektrische Filter 40 zugeordnet ist. In diesem Fall muss der jeweilige elektrische Filter 40 mit demjenigen der Leiter 26 elektrisch verbunden werden, der zu einer benachbarten elektrisch leitenden Hülle führt und nicht zu derjenigen elektrisch leitenden Hülle, innerhalb der der elektrische Filter 40 angeordnet ist. Die Herstellung einer derartigen Elektrodenleitung ist in den Figuren 7 bis 14 näher beschrieben.

Wie weiter vorne bereits erläutert, kann ein jeweiliger elektrischer Filter 40 für einen proximalen Elektrodenpol auch proximal dieses Elektrodenpols angeordnet werden, da in diesem Bereich eine Elektrodenleitung typischerweise auch alternative versteifende Stützelemente zum Stabilisieren des Filters angeordnet werden können.

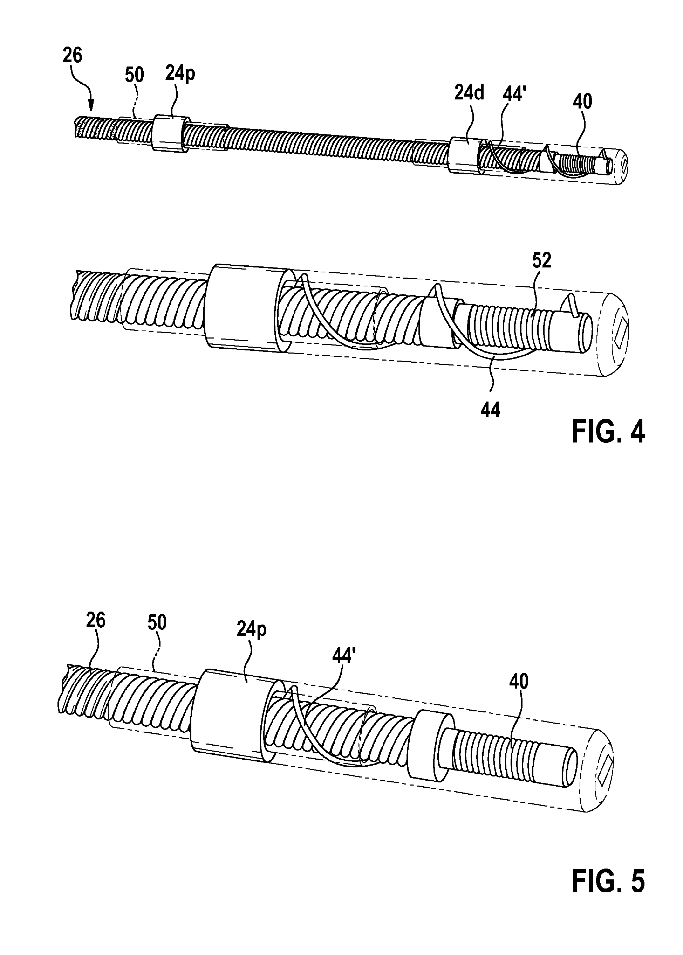

Ähnlich ist es auch im Falle eines distalsten Ringelektrodenpols 24 möglich, den zugehörigen elektrischen Filter 40 nicht innerhalb der den distalsten Ringelektrodenpol bildenden elektrisch leitenden Hülle anzubringen, sondern an einem weiter distal gelegenen Ort der Elektrodenleitung 20. Dazu wird ein entsprechender Leiter 26, der im Falle einer Elektrodenleitung die elektrische Hülle des distalen Ringelektrodenpols direkt kontaktieren könnte, einige Millimeter weitergeführt und mit einer (proximalen) Anschlusshülle 42 des entsprechenden elektrischen Filters 40 kontaktiert. Die entsprechende distale Anschlusshülle 46 des elektrischen Filters 40 wird schließlich über einen zur elektrischen Hülle zurückgeführten Draht 44', zum Beispiel über ein locker gewendeltes Seil, mit der elektrisch leitenden Hülle verbunden; siehe Fig. 4.

Bei dem in Fig. 4 dargestellten Beispiel weist der elektrische Filter 40 einen Draht 52 auf, der einlagig gewickelt ist und eine Spule damit eine Induktivität des elektrischen Filters 40 bildet. Wenn der die Spule und damit die Induktivität des elektrischen Filters bildende Draht nicht einlagig (oder mit einer ungeraden Anzahl von Lagen) gewickelt wird, sondern mit einer geraden Anzahl von Lagen, können beide Anschlusshüllen des elektrischen Filters beispielsweise an dessen proximalem Ende vorgesehen sein, so dass insbesondere der Draht 44' nur über eine kürzere Distanz zurückgeführt werden muss; siehe Fig. 5.

Bei einer zweipoligen Elektrodenleitung, bei der die beiden Elektrodenpole jeweils von Ringelektroden gebildet werden, kann ein elektrischer Filter für den dann distalen Elektrodenpol so angeordnet werden, wie dies in den Figuren 4 oder 5 gezeigt ist. Der elektrische Filter für den proximalen Elektrodenpol kann proximal desselben angeordnet werden, wie dies zuvor erwähnt wurde. In diesem Fall ist kein elektrischer Filter innerhalb einer elektrisch leitenden Hülle angeordnet.

Bei allen Ausführungsvarianten können gegebenenfalls einzelne Leiter aus der Coradialwendelzuleitung herausgedreht und von ihrer Isolation befreit werden. In diesem Fall entstehen Windungslücken in der verbleibenden Coradialwendelzuleitung. Um nicht-isolierte Leiter in diesem Fall weiter axial entlang der Coradialwendelzuleitung zu führen ist es vorteilhaft, wenn die zunächst verbleibende Coradialwendelzuleitung mit ihren Windungslücken mit einer isolierenden Schicht, zum Beispiel einem Silikonschlauch, bezogen wird und anschließend die nicht-isolierten Leiter in die Windungslücken hineingewickelt werden, wie dies in Fig. 6 dargestellt ist.

Ein zusätzlicher Stützschlauch (zum Beispiel aus Polyimid) kann im Inneren der Coradialwendelzuleitung angebracht werden. Auf diese Weise ergibt sich eine mechanisch belastbare und dennoch flexible Coradialwendelzuleitung, die vielfältig angepasst werden kann und insbesondere beispielsweise dafür benutzt werden kann, um einen distal gelegenen elektrischen Filter, wie er in den Figuren 4 und 5 dargestellt ist, zu kontaktieren.

Figuren 7 bis 14 illustrieren, wie eine Elektrodenleitung gemäß einer vorteilhaften Ausführungsvariante hergestellt werden kann, bei der ein jeweiliger elektrischer Filter innerhalb einer elektrisch leitenden Hülle angeordnet ist, die derjenigen elektrisch leitenden Hülle benachbart ist, welche den Elektrodenpol bildet, dem der elektrische Filter zugeordnet ist und bei der der elektrische Filter für den proximalsten Elektrodenpol proximal desselben angeordnet ist.

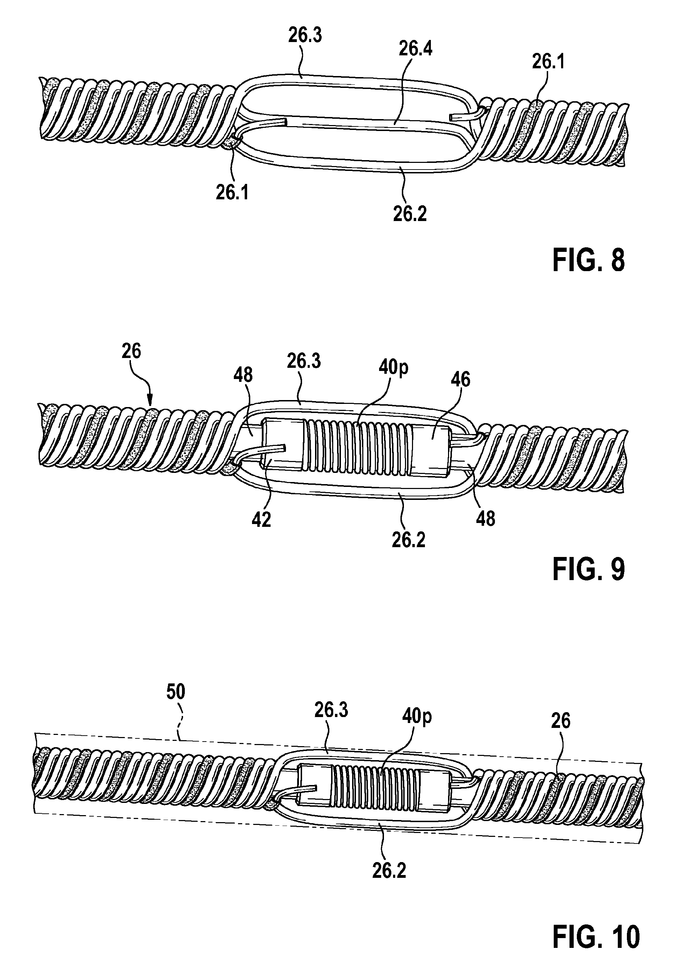

Fig. 7 zeigt einen Abschnitt der fertigen Elektrodenleitung 20 von außen. Zu erkennen ist eine proximaler Ringelektrodenpol 24p und ein distaler Ringelektrodenpol 24d. Beide werden von einer jeweiligen elektrisch leitenden Hülle, beispielsweise einer Metallhülle, gebildet. Zu erkennen ist auch, wie die Elektrodenleitung 20 zwischen den Elektrodenpolen und diesseits und jenseits der Elektrodenpole eine elektrisch isolierende Hülle 50 trägt, die in dem Ausführungsbeispiel von einem Silikonschlauch gebildet ist. Innerhalb dieser Hülle 50 und innerhalb der elektrisch isolierenden Hüllen 24p und 24d erstreckt sich eine Coradialwendelzuleitung 26, die insbesondere von vier elektrischen Leitern 26.1, 26.3, 26.3 und 26.4 (siehe auch Figur 8) gebildet ist. Proximal des proximalen Elektrodenpols 24p ist der dazugehörige elektrische Filter 40p angeordnet. Der elektrische Filter 40d für den distalen Elektrodenpol 24d ist innerhalb der elektrisch leitenden Hülle des proximalen Elektrodenpols 24p angeordnet.

Zur Erleichterung des Verständnisses ist einer der vier Leiter 26.1, 26.2, 26.3 und 26.4 der Coradialwendelzuleitung 26 dunkel dargestellt, während die übrigen drei Leiter hell dargestellt sind.

Die Figuren 8 und 9 erläutern zunächst den Einbau des proximalen elektrischen Filters 40p. Zunächst wird die Coradialwendelzuleitung 26 dort, wo der proximale elektrische Filter 40p angeordnet werden soll, aufgedreht und der dem proximalen Elektrodenpol 24p zugeordnete elektrische Leiter 26.1 aufgetrennt. Die übrigen elektrischen Leiter 26.2, 26.3 und 26.4 werden nicht aufgetrennt und bleiben isoliert. Die aufgetrennten Enden des Leiters 26.1 werden von ihrer Isolierung befreit; siehe Fig. 8.

Anschließend kann der elektrische Filter 40p an dem durch das Aufdrehen der Windungen frei gewordenen Ort eingesetzt werden. Hierzu wird zunächst der elektrische Filter 40p in den Raum eingelegt und anschließend ein Polyimidschlauch 48 durch das Lumen der Coradialwendelzuleitung 26 und des elektrischen Filters 40p hindurchgeführt, um den elektrischen Filter 40p zuverlässig und stabil an seinem Ort zu halten. Daraufhin werden die von der Isolation befreiten freien Drahtenden des aufgetrennten Leiters 26.1 mit der proximalen Anschlusshülle 42 und der distalen Anschlusshülle 46 des elektrischen Filters 40p verschweißt und auf diese Weise elektrisch verbunden; siehe Fig. 9.

Anschließend wird die elektrisch isolierende Hülle 50 in Form eines Silikonschlauchs über die so hergestellte Baugruppe geschoben.

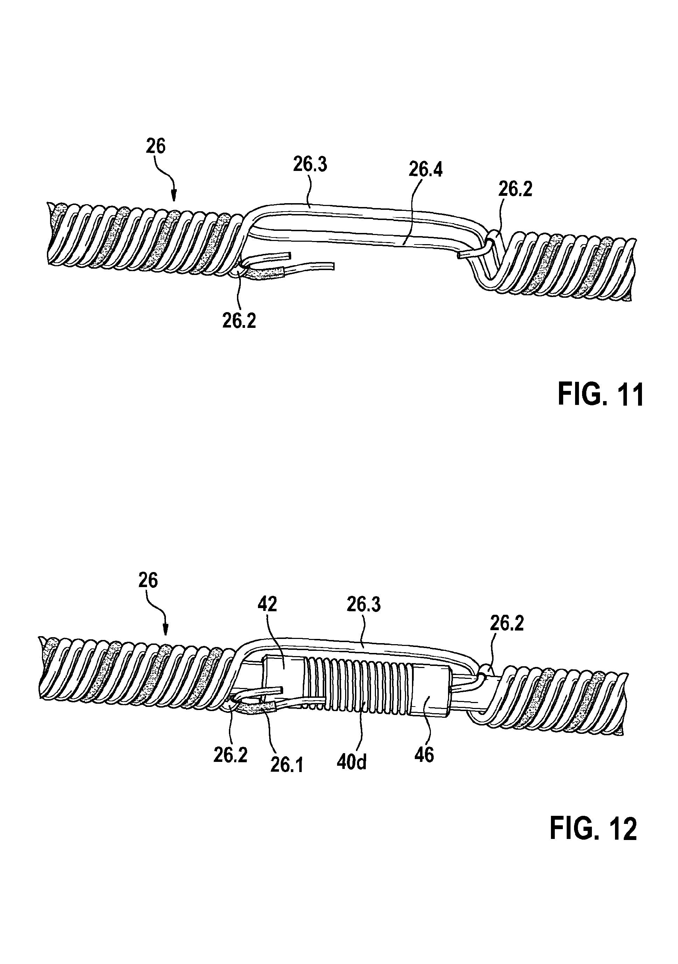

Für den Einbau des distalen elektrischen Filters 40d wird die Coradialwendelzuleitung 26 am Ort des proximalen Elektrodenpols 24p aufgedreht und ein weiterer Leiter 26.2 der Coradialwendelzuleitung 26 wird aufgetrennt und die aufgetrennten Enden werden von ihrer Isolation befreit. So ergibt es sich, dass am Ort des proximalen Elektrodenpols 24p zwei der Leiter der Coradialwendelzuleitung ungetrennt durchlaufen, während der Leiter 26.1 zum Kontaktieren der elektrisch leitenden Hülle des proximalen Elektrodenpols 24.p mit einem nicht-isolierten Ende vorhanden ist und der Leiter 26.2 zum Kontaktieren des distalen Elektrodenpols 24d im Bereich des proximalen Elektrodenpols zwei aufgetrennte und von Isolation befreite Leitungsenden zeigt; siehe Fig. 11.

Wie schon beim zuvor beschriebenen proximalen elektrischen Filter 40p wird nun der distale elektrische Filter 40d in den durch Aufdrehen der Coradialwendelzuleitung 26 entstandenen Raum eingesetzt und dessen Anschlusshüllen 42 und 46 werden durch Schweißen mit den freien und von Isolation befreiten aufgetrennten Enden des Leiters 26.2 elektrisch verbunden; siehe Fig. 12.

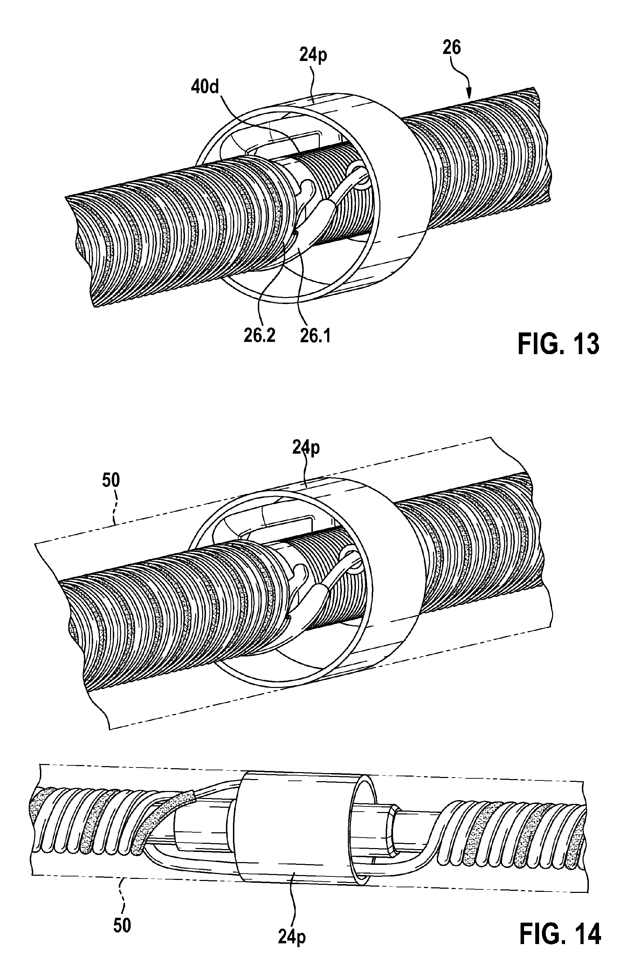

Anschließend kann die den proximalen Elektrodenpol 24p bildende elektrisch leitende Hülle bis direkt über den elektrischen Filter 40d auf die Coradialwendelzuleitung 26 aufgeschoben werden; siehe Fig. 12 und 13. Das von Isolation befreite freie Ende des Leiters 26.1 (der dunkle Leiter) kann mit der entsprechenden elektrisch leitenden Hülle 24p verbunden werden; siehe Fig. 12.

Derjenige Teil des Leiters 26.1 (des in der Figur dunkel dargestellten Leiters), der sich distal des proximalen Elektrodenpols 24p erstreckt, ist nicht weiter elektrisch kontaktiert und dient ausschließlich dazu, dass in der weiteren Coradialwendelzuleitung 26 keine Windungslücken entstehen.

Um elektrische Kurzschlüsse zu vermeiden, müssen alle nicht-isolierten Drahtabschnitte und offenliegenden Kontakte im Bereich der elektrisch leitenden Hülle des proximalen Elektrodenpols 24p voneinander isoliert werden, beispielsweise durch eine entsprechende Parylene-Beschichtung, eine Sprühbeschichtung oder durch Ausspritzen des Bereichs mit Silikon. Anschließend können weitere Teile der elektrisch isolierenden Außenhülle 50 aufgebracht oder aufgestreift werden, um auf diese Weise die Elektrodenleitung 20 fertigzustellen, wie in Fig. 14 dargestellt.

### Bezugszeichenliste

10 - Implantierbarer Herzstimulator
12- Gehäuse
14 - Anschlussgehäuse
16 - Kontakte
20 - Elektrodenleitung
22 - Spitzen- oder Tipelektrode
24 - Ringelektrode
22, 24 - Elektrodenpole
26.1, 26.2, 26.3, 26.4 - Elektrische Leiter
26 - Coradialwendelzuleitung
26.1, 26.3, 26.3 und 26.4 - Elektrischen Leiter
26.2 - Leiter
28 - Steckkontakt
40 - Elektrischer Filter
42 - Proximale Anschlusshülle
44 - Draht
46 - Distale Anschlusshülle
48 - Hilfskörper
50 - Elektrisch isolierende Hülle
52 - Draht

## Patentansprüche

1. Implantierbare elektrische Leitung (20) mit wenigstens einem helixartig gewendelten elektrischem Leiter (26), einer mit dem elektrischen Leiter elektrisch verbundenen elektrisch leitenden Hülle (22; 24) und einem elektrischen Filter (40), **dadurch gekennzeichnet, dass** der elektrische Filter in Längsrichtung der elektrischen Leitung (20) gesehen zwischen einem proximalen (20p) und einem distalen Längsabschnitt (20d) einer von dem helixartig gewendelten elektrischen Leiter (26) gebildeten Helix sowie in radialer Richtung der elektrischen Leitung (20) gesehen innerhalb der elektrisch leitenden Hülle (22; 24) angeordnet ist.

2. Implantierbare elektrische Leitung (20) nach Anspruch 1, **dadurch gekennzeichnet, dass** die implantierbare elektrische Leitung (20) eine mehrpolige Elektrodenleitung mit mehreren elektrisch leitenden Hüllen (22, 24) als Elektrodenpolen ist, die jeweils mit einem elektrischen Leiter (26) elektrisch verbunden sind, wobei die elektrischen Leiter eine coradiale Leitungswendel bilden.

3. Implantierbare elektrische Leitung (20) nach Anspruch 1 oder 2, **dadurch gekennzeichnet, dass** ein elektrischer Filter für einen proximalsten Elektrodenpol proximal der dazugehörigen elektrisch leitenden Hülle angeordnet ist.

4. Implantierbare elektrische Leitung (20) nach mindestens einem der Ansprüche 1 bis 3, **dadurch gekennzeichnet, dass** der elektrische Filter für den distalsten Elektrodenpol distal zu diesem Elektrodenpol angeordnet ist.

5. Implantierbare elektrische Leitung (20) nach Anspruch 1 oder 2, **dadurch gekennzeichnet, dass** der elektrische Filter innerhalb derjenigen elektrisch leitenden Hülle angeordnet ist, die denjenigen Elektrodenpol bildet, dem dieser elektrische Filter zugeordnet ist.

6. Implantierbare elektrische Leitung (20) nach mindestens einem der Ansprüche 1 bis 5, **dadurch gekennzeichnet, dass** eine jeweilige elektrisch leitende Hülle jeweils eine Ringelektrode der Elektrodenleitung bildet.

7. Implantierbare elektrische Leitung (20) nach mindestens einem der Ansprüche 1 bis 6, **dadurch gekennzeichnet, dass** der elektrische Filter eine Spule aus isoliertem Draht aufweist, der mit einer ungeraden Anzahl an Lagen um einen hohlen, zylindrischen Kern helixförmig gewickelt ist.

8. Implantierbare elektrische Leitung (20) nach mindestens einem der Ansprüche 1 bis 6, **dadurch gekennzeichnet, dass** der elektrische Filter eine Spule aus isoliertem Draht aufweist, der mit einer geraden Anzahl an Lagen um einen hohlen, zylindrischen Kern helixförmig gewickelt ist.

9. Implantierbare elektrische Leitung (20) nach mindestens einem der Ansprüche 1 bis 6, **dadurch gekennzeichnet, dass** der elektrische Filter einen einlagig gewickelten, nicht-isolierten oder isolierten Draht und optional ein zusätzliches kapazitives Element aufweist.

10. Implantierbare elektrische Leitung (20) nach mindestens einem Ansprüche 1 bis 6, **dadurch gekennzeichnet, dass** der elektrische Filter eine spiralförmig um einen hohlen, zylindrischen Kern gewickelte Spule aus einseitig metallisierter Folie aufweist.

11. Implantierbare elektrische Leitung (20) nach Anspruch 10, **dadurch gekennzeichnet, dass** die Folie eine Kunststofffolie ist.

12. Implantierbare elektrische Leitung (20) nach mindestens einem der Ansprüche 1 bis 10, **dadurch gekennzeichnet, dass** der elektrische Filter ein Bandstopp- oder Tiefpass-Filter ist.

13. Implantierbare elektrische Leitung (20) nach mindestens einem der Ansprüche 1 bis 10, **dadurch gekennzeichnet, dass** ein jeweiliger elektrischer Filter (40) mit aufgetrennten Leitungsenden eines aufgetrennten Leiters (26.1) einer Coradialwendelzuleitung (26) elektrisch verbunden ist und andere Leiter (26.2, 26.3, 26.4) der Coradialwendelzuleitung (26) nicht aufgetrennt, sondern ununterbrochen an dem elektrischen Filter vorbeigeführt sind.

14. Herstellungsverfahren für eine Elektrodenleitung (20) mit wenigstens einem Elektrodenpol (24) und einem elektrischen Filter (40), **gekennzeichnet durch** die Schritte
Aufdrehen der Coradialwendelzuleitung (26) am Ort des Elektrodenpols (24),
Auftrennen eines Leiters (26.1) der Coradialwendelzuleitung (26), so dass aufgetrennte Leitungsenden entstehen,
Befreien der aufgetrennten Leitungsenden von Isolation,
Einsetzen eines elektrischen Filters (40) dort, wo die Coradialwendelzuleitung (26) aufgedreht ist,
elektrisches Kontaktieren des elektrischen Filters (40) mit den von Isolation befreiten Enden des aufgetrennten Leiters,
Aufschieben einer einen späteren Elektrodenpol bildenden elektrisch leitenden Hülle, so dass sich der elektrische Filter (40) schließlich innerhalb der elektrisch leitenden Hülle befindet,
Kontaktieren der elektrisch leitenden Hülle,
Isolieren offenliegender Drähte und Kontaktstellen im Bereich des Elektrodenpols.
